# EUROPEAN PATENT SPECIFICATION

(11) **EP 4 519 652 B1**
(45) Date of publication and mention of the grant of the patent: **18.02.2026**
(21) Application number: 22741366.3
(22) Date of filing: 06.05.2022
(51) Int. Cl.: G01N 13/00, G01N 15/08, G01N 1/28, G01N 33/24

(54) **METHOD FOR PREPARING A ROCK SAMPLE WITH A TARGET INITIAL FIRST FLUID SATURATION PROFILE ALONG A ROCK SAMPLE AXIS**
VERFAHREN ZUR HERSTELLUNG EINER GESTEINSPROBE MIT EINEM ERSTEN ZIELFLÜSSIGKEITSSÄTTIGUNGSPROFIL ENTLANG EINER GESTEINSPROBENACHSE
PROCÉDÉ DE PRÉPARATION D'UN ÉCHANTILLON DE ROCHE AVEC UN PREMIER PROFIL DE SATURATION DE FLUIDE INITIAL CIBLE LE LONG D'UN AXE D'ÉCHANTILLON DE ROCHE

(43) Date of publication of application: 12.03.2025
(73) Proprietor: TotalEnergies OneTech, 92400 Courbevoie (FR)
(72) Inventor: DE OLIVEIRA FERNANDES, Victor, 64018 Pau Cedex (FR); CAUBIT, Cyril, 64018 Pau Cedex (FR); NICOT, Benjamin, 64000 Pau (FR); PAIROYS, Fabrice, 64018 Pau Cedex (FR)
(74) Representative: Lavoix
(86) International application number: PCT/IB2022/000267
(87) International publication number: WO 2023/214192

(56) References cited:
- US-A- 5 493 226
- US-A1- 2005 240 360
- US-A1- 2016 109 334

## Description

The invention relates to a method for preparing a rock sample with a target initial first fluid saturation profile along a rock sample axis.

In the domain of oil and gas exploration, it is critical to define the properties of rocks that are located at an exploration site, since local properties to the rock impact the overall behavior of the explored site. Such properties can include, for example, relative permeability or wettability of the rock.

In order to define properties of the rock, a rock sample is in general extracted from the exploration site and several tests are conducted of the rock sample, in order to define the rock properties.

In order to properly characterize the properties of the rock, and incidentally, the behavior of the exploration site, it is generally required to prepare the rock sample so that it exhibits a target initial first fluid saturation profile along a rock sample axis in agreement with logging results. Such initial first fluid saturation profile is generally an initial water saturation profile, often referred to as "Swi", and correspond to a proportion of water relatively to the overall fluid present in the rock (for example mix of the first fluid with a second fluid such as oil), along the rock sample axis. The target first fluid saturation profile is often chosen so that it corresponds to the first fluid saturation profile of the rock extracted on the exploration site.

A first method for preparing a rock sample with a target initial first fluid saturation profile along a rock sample axis consists in saturating the rock sample with the first fluid, before flooding the rock sample with a second fluid from an inlet face, and retrieving a production of both first and second fluids from a channel directly connected to an outlet of the rock sample. Such method allows preparing a rock sample with a target initial first fluid saturation profile quickly but is not entirely satisfying since the obtained first fluid saturation profile is not homogenous along the rock sample axis and the correct values of first fluid saturation are not well defined.

A second method to overcome the above-mentioned drawback has thus been proposed. As for the first method, this second method involves saturating the rock sample with the first fluid, before flooding the rock sample with a second fluid from an inlet face. However, in this method only the first fluid is retrieved from a channel connected to the outlet of the rock sample through a porous barrier element. This method allows obtaining a first fluid saturation profile that is homogenous along the rock sample axis and has a good reliability on setting correct first fluid saturation, but this method is also unsatisfying, since the duration for obtaining the target initial first fluid saturation profile is too long to comply with the exploration requirements.

Patent application US2016/109334 A1 discloses a rock flood testing method for injecting injection fluid into a plurality of rock samples, the method comprising: arranging the plurality of rock samples in respective ones of a corresponding plurality of pressure vessels, wherein the rock samples comprise oil and water at an initial water saturation, ageing the rock samples such that the samples are in a mixed wettability state; injecting an injection fluid into each of the rock samples, removing fluid displaced from the rock samples, analysing fluids displaced from each of the rock samples.

A goal of the invention is thus to solve the drawbacks of both above mentioned methods, by having a method that allows rapidly obtaining a first fluid saturation profile that is homogenous along the rock sample axis.

To that end, the invention relates to a method for preparing a rock sample according to claim 1.

Such method allows quickly flooding the rock sample with the primary flooding and then adjusting the fluid saturation profile so that it becomes homogenous and compliant with the target initial first fluid saturation profile along the rock sample axis, resulting in a rapid preparation of a rock sample with a homogenous initial first fluid saturation profile along the rock sample axis that correspond to the target profile.

According to specific embodiments of the invention, the method further presents one or several of the features corresponding to claims 2 to 12.

Other features and advantages of the invention will become apparent from a detailed description which is given thereof below, as an indication and by no means as a limitation, with reference to the appended figures, wherein:
- Figure 1 is a schematic representation of a rock properties measurement system according to the invention;
- Figure 2 is a schematic representation of the rock properties measurement cell of the rock properties measurement system of figure 1;
- Figure 3 is a graph of an evolution of the first fluid saturation profile during a primary flooding implemented in the rock properties measurement cell of figure 2;
- Figure 4 is a graph of an evolution of the first fluid saturation profile during a secondary flooding implemented in the rock properties measurement cell of figure 2; and

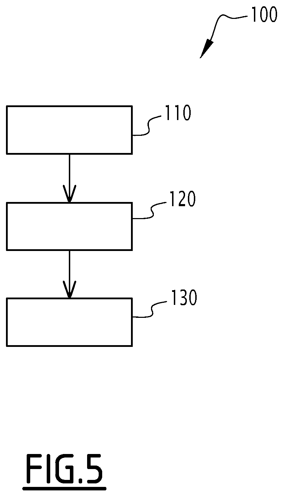

- Figure 5 is an organigram of the steps of a method for preparing a rock sample with a target initial first fluid saturation profile along a rock sample axis, implemented by the rock properties measurement system of figure 1.

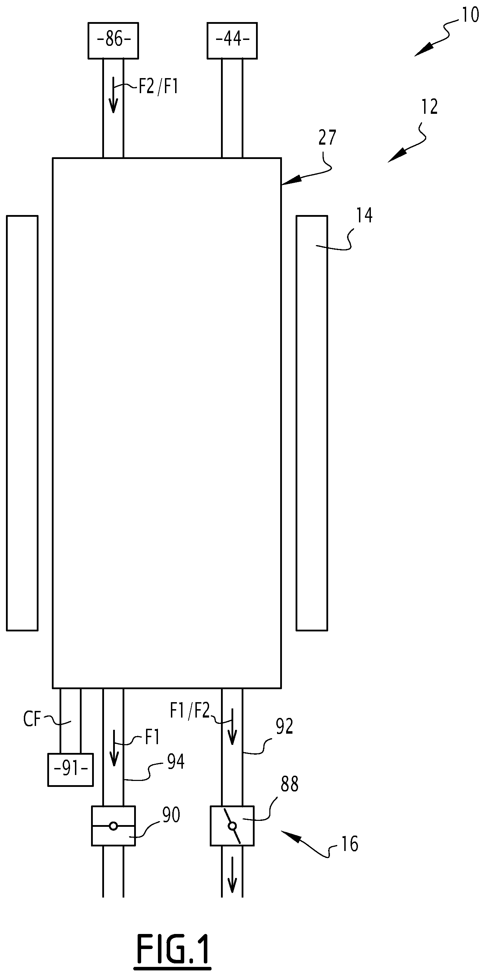

Referring to figure 1, a rock properties measurement system 10 comprises a rock properties measurement cell 12, an inner structure imaging device 14 and a fluid regulation arrangement 16.

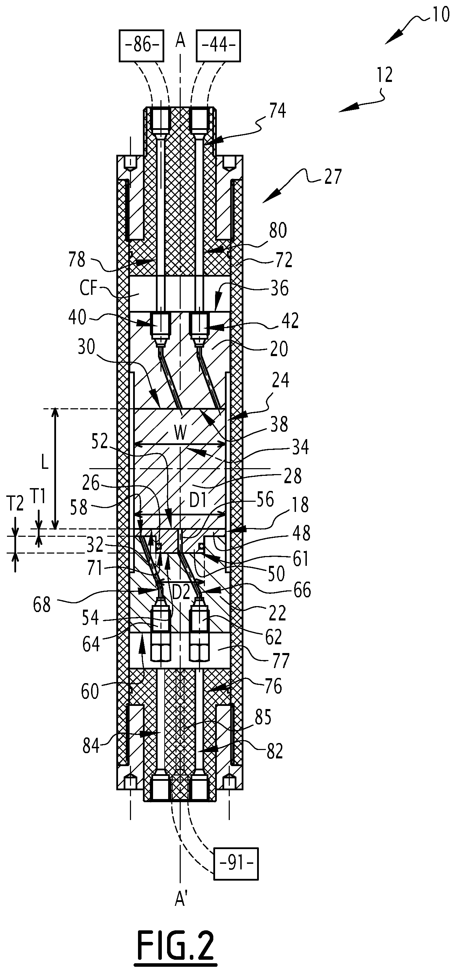

An example of the rock properties measurement cell 12 is illustrated on figure 2. The rock properties measurement cell 12 comprises a monolithic porous barrier element 18, an upstream diffuser 20, a downstream diffuser 22 and a sleeve 24.

The rock properties measurement cell 12 further preferably comprise a sealing gasket 26 and a confinement housing 27.

The rock properties measurement cell 12 is configured for receiving a rock sample 28 to carry out inner structure imaging of at least a fluid F1, F2, contained in said rock sample 28.

The rock properties measurement cell 12 is in particular configured for receiving a rock sample 28 to carry out measurement of a saturation profile S1i of a first fluid in in the rock sample.

In the rest of the description, the terms upstream and downstream are understood by reference with the displacement of the at least a fluid F1, F2, within the rock properties measurement cell 12, that is an upstream element being located higher on the figure 2 than a downstream element.

As this will be exposed later, the first fluid F1 is preferably water and/or the second fluid is preferably oil.

In particular, the first fluid is preferably water including at least one salt such as a brine with a salt concentration comprised between 1 g/l and 300 g/l. The water including at least one salt may comprise a plurality of salts of different compositions.

The second fluid is for example an oil or a gas chosen from the following types of oils and gas : mineral synthetic oil, dead oil, live oil, Nitrogen, compressed air, Helium.

In alternative, and for example, the first fluid F1 is oil and/or the second fluid F2 is water.

It will be understood that first F1 and/or second F2 fluids can also be other fluids differing from water and oil.

As seen on figure 2, the upstream diffuser 20, the rock sample 28, the porous element 18 and the downstream diffuser 22 are aligned in this order in a flow direction of the at least one fluid F1, F2.

The rock sample 28 is of any type and is for example a rock sample that is extracted from an oil and gas exploration site. In some specific examples, the permeability of the rock sample is low, that is the permeability of the rock sample is under 1 mD (milliDarcy).

The rock sample 28 defines a rock sample axis A-A' and is preferably elongated along the rock sample axis A-A'.

The rock sample 28 comprises an inlet face 30 and an outlet face 32. The inlet face 30 and the outlet 32 face are opposed relatively to the rock sample axis A-A' and are transverse to the rock sample axis A-A'.

In the example of figure 2, the rock sample 28 is cylindrical.

A length L of the rock sample 28 along the rock sample axis A-A' is for example comprised between 15 mm and 300 mm.

The width W of the rock sample 28, taken transversally to the rock sample axis A-A', is for example comprised between 10mm and 75 mm

As illustrated in figure 2, the rock sample 28 is received in measurement chamber 34 of the measurement cell 12.

The measurement chamber 34 is delimited by the upstream diffuser 20, the porous barrier element 18 and the sleeve 24.

The dimension of the measurement chamber 34 is for example similar to the dimensions of the rock sample 28. The rock sample 28 is thus for example in contact with the upstream diffuser 20, the porous barrier element 18 and the sleeve 24 when the rock sample 28 is received in the measurement chamber 34.

The sleeve 24 is arranged around the upstream diffuser 20, the downstream diffuser 22 and the porous barrier element 18. As illustrated in figure 1, the sleeve 24 is arranged around the porous barrier element 18 to surround the entire porous barrier element 18 along the rock sample axis A-A' and is arranged to at least partially surround the upstream diffuser 20 and the downstream diffuser 22.

The sleeve 24 is preferably arranged to surround the rock sample 28 along the rock sample axis A-A'. In the example of figure 2 where the rock sample 28 is cylindrical, the sleeve 24 is a tubular sleeve 24.

The inner diameter of the sleeve 24 therefore sensibly corresponds to the width W of the rock sample 28, the inner diameter of the sleeve 24 being inferior or equal to the width W of the rock sample 28.

For example, the inner diameter of the sleeve 24 is equal to 8 mm when the width W of the rock sample 28 is equal to 10 mm, and is equal to 36 mm when the width W of the rock sample 28 is equal to 38 mm, the elasticity of the sleeve 24 allowing the sleeve to be arranged around the rock sample 28.

The sleeve 24 is arranged to surround the barrier element 18.

As seen on figure 2 the sleeve 24 preferably protrudes on both sides of the rock sample 28 along the rock sample axis A-A' so that is also surround at least a partially the upstream diffuser 20 and the downstream diffuser 22.

A thickness of the sleeve 24 is comprised between 0.5mm and 5mm and is for example equal to 1 mm.

The sleeve 24 is transparent to inner structure imaging such as microtomography imaging and/or nuclear magnetic resonance imaging.

The sleeve 24 is impervious to the fluids present in the rock sample F1, F2 so that the sleeve 24 guides the fluids F1 and/or F2 along the rock sample axis A-A' within the rock sample 28.

The sleeve 24 is for example made of fluorocarbon-based fluoroelastomer. The sleeve 24 is for example made of Viton^{®}, a fluorocarbon-based fluoroelastomer developed by the company DuPont.

As it will be presented later, the sleeve 24 is for example pressed around the upstream diffuser 20, the downstream diffuser 22, the porous barrier element 18 and the rock sample 28 received in the measurement chamber 34 when the measurement cell 12 is used to measure rock properties.

The upstream diffuser 20 is aligned to the rock sample axis and is located upstream of the measurement chamber 34, or in other terms, upstream of the rock sample 28.

The upstream diffuser 20 is preferably transparent to inner structure imaging such as microtomography imaging and/or nuclear magnetic resonance imaging.

The upstream diffuser 20 is for example made of a poly ketone polymer, in particular polyether ether ketone.

The upstream diffuser 20 is configured to guide at least second fluid to the rock sample 28.

The upstream diffuser 20 comprises an upstream side 36 and a downstream side 38 opposed to the upstream side 36 relatively to the upstream diffuser 20.

The upstream diffuser 20 also comprises a first upstream opening 40 and comprises for example a second upstream opening 42.

The first upstream opening 40 extends through the upstream diffuser 20 to connect the downstream side 38 and the upstream side 36 of the upstream diffuser 20.

The first upstream opening 40 is configured for guiding the second fluid F2 into the rock sample from the upstream side 36 to the downstream side 38 of the upstream diffuser 20.

In the example of figure 2, the first upstream opening 40 is angled relatively to the rock sample axis A-A'. The angle defined between the first upstream opening 40 and the rock sample axis is for example comprised between 0° and 45°, for example between 5° and 30° .

The second upstream opening 42 extends through the upstream diffuser 20 to connect the downstream side 38 and the upstream side 36 of the upstream diffuser 20.

The second upstream opening 42 is configured for fluidically connecting the rock sample with a pressure measurement device 44 of the fluid regulation arrangement 16.

In the example of figure 2, the second upstream opening 42 is angled relatively to the rock sample axis A-A' similarly to the first upstream opening 42.

In a particular example, the downstream side 38 of the upstream diffuser 20 comprises at least one groove (not shown) connected to the first upstream opening 40. The groove extends for example around the rock sample axis A-A' and is configured to guide the second fluid F2 along the inlet face 30 of the rock sample 28.

As shown on figure 2, a downstream portion of the diffuser has a smaller radial section than an upstream portion of the upstream diffuser 20. The downstream portion of the upstream diffuser 20 is for example surrounded by the sleeve 24.

The monolithic porous barrier element 18 is aligned to the rock sample axis A-A' and is located downstream of the measurement chamber 34, or in other terms, downstream of the rock sample 28.

The monolithic porous barrier element 18 is for example made of ceramic and for example an alpha alumina ceramic.

Pores size of the monolithic porous barrier element 18 is for example comprised between 20nm and 20nm and is for example equal to 50nm, 80nm or 150nm.

The monolithic porous barrier element is for example formed from an oil wet ceramic or a water wet ceramic.

The monolithic porous barrier element 18 is permeable to the first fluid F1 and impervious to the second fluid F2. In other words, when a first fluid F1 and a second fluid F2 are located within the rock sample 28, only the first fluid F1 can be retrieved from the rock sample 28 through the porous barrier element 18.

The monolithic porous barrier element 18 comprises an upstream segment 48 of a first diameter D1 and a downstream segment 50 of a second diameter D2. The second diameter D2 is smaller than the first diameter D1. In a median cross section, the upstream segment 48 and the downstream segment 50 define a T shape of the monolithic porous barrier element 18.

The upstream segment 48 defines an upstream face 52 of the rock sample. As visible on figure 2, the upstream face 52 is configured to cooperate with the rock sample 28, in particular such that the outlet face 32 of the rock sample 28 is in contact with the upstream face 52 of the porous barrier element 18.

As visible on figure 2, the upstream face 52 is for example essentially flat. Alternatively, and for example, the upstream face 52 is substantially flat and comprises at least one groove (not illustrated).

The upstream 48 and downstream 50 segments define together a downstream face 54 opposed to the upstream face 52. As shown on figure 2, the downstream face 54 therefore comprise two annular sub-faces connected together by a cylindrical sub-face.

The porous barrier element 18 comprises a bypass 56 connecting the upstream face 52 and the downstream face 54 through both the upstream segment 48 and the downstream segment 50. Thus, when a first fluid F1 and a second fluid F2 are located within the rock sample 28, both the first fluid F1 and the second fluid F2 can be retrieved from the rock sample 28 through the bypass 56.

In the example presented in figure 2, the porous barrier element 18 is a revolution element and the bypass 56 extends through the porous barrier element 18 following the axis of revolution of the element. In particular, the bypass 56 is aligned to the rock sample axis A-A'.

The first diameter D1 preferably corresponds to the width W of the rock sample axis. The thickness T1 of the upstream segment is preferably comprised between 3mm and 20mm.

The ratio of the thickness T1 over the diameter D1 of the upstream segment 48 is for example smaller than 40%.

The thickness T2 of the downstream segment is preferably comprised between 5mm and 50mm.

The downstream diffuser 22 is aligned to the rock sample axis A-A' and is located downstream of the porous element barrier element 18.

The downstream diffuser 22 is preferably transparent to inner structure imaging such as microtomography imaging and/or nuclear magnetic resonance imaging.

The downstream diffuser 22 is for example made of a poly ketone polymer, in particular polyether ether ketone.

The downstream diffuser 22 comprises an upstream side 58 and a downstream side 60 opposed to the upstream side 56 relatively to the downstream diffuser 22.

The downstream side 60 of the downstream diffuser 22 is for example essentially flat. Alternatively, and for example, the downstream side 60 of the downstream diffuser 22 is substantially flat and comprises at least one groove (not illustrated).

As illustrated in figure 2, the downstream diffuser 22 defines a cavity 61.

The cavity 61 is delimited by the upstream side 58 of the downstream diffuser 22. As shown on figure 2, the upstream side 58 therefore comprise two annular sub-faces connected together by a cylindrical sub-face.

The cavity 61 is conformed to receive the porous barrier element 18. In particular, and as shown on figure 2, the downstream segment 50 of the porous element 18 is received in the cavity 61, the shape of the cavity 61 being complementary to the shape of the downstream segment 50 of the porous element 18.

The downstream diffuser 22 comprises a first downstream opening 62 and a second downstream opening 64.

The first downstream opening 62 is for example aligned with the bypass, that is, the first downstream opening 62 is connected to the bypass 56. The first downstream opening 62 delimits together with the bypass 56 a first open channel 66.

In the example of figure 2, the first downstream opening 62 is angled relatively to the rock sample axis A-A'. The angle defined between the first downstream opening 62 and the rock sample axis is for example comprised between 0° and 45°, for example between 5° and 30°.

The first open channel 66 opens directly on the rock sample 28 and extends through the downstream diffuser 22 and the barrier element 18.

The first open channel 66 is configured to retrieve first F1 and/or second F2 fluid from the rock sample 28.

The second downstream opening 64 forms a second open channel 68.

In the example of figure 2, the second downstream opening 64 is angled relatively to the rock sample axis A-A' similarly to the first downstream opening 62.

The second open channel 68, opens facing the porous barrier element 18 and in particular facing the upstream segment 48 on the downstream face 54.

The second open channel 68 in configured to retrieve first fluid from the rock sample 28. In particular, the second open channel 68 is configured to retrieve only the first fluid F1 from the rock sample 28 that passed through the porous barrier element 18.

As presented in figure 2, the sealing gasket 26 is arranged around the downstream segment 50 of the porous element 18.

The sealing gasket 26 is arranged between, preferably radially between, the downstream segment 50 and the downstream diffuser 22.

In a preferred embodiment, the porous element comprises an annular peripheral housing 71 the sealing gasket 26 being arranged in the annular peripheral housing. In a non-illustrated embodiment, the sealing gasket 26 is instead arranged in an annular peripheral housing of the downstream diffuser 22.

As illustrated in figure 2, the confinement housing 27 is arranged around the sleeve 24. Since the sleeve 24 is arranged at least partially around the upstream diffuser 20, the downstream diffuser 22 and the porous barrier element 18, the confinement housing 27 is also arranged around the upstream diffuser 20, the downstream diffuser 22 and the porous barrier element 18, and around the sample 28 received in the measurement chamber 34

The confinement housing 27 is transparent to inner structure imaging such as microtomography imaging and/or nuclear magnetic resonance imaging.

The confinement housing 27 is for example made of a poly ketone polymer, in particular polyether ether ketone.

The confinement housing 27 comprises a confinement frame 72, a upstream cap 74 and a downstream cap 76.

The confinement housing 27 is configured to receive a confinement fluid CF to apply pressure to the sleeve 24 around the upstream diffuser 20, the downstream diffuser 22, the porous barrier element 18 and the rock sample 28 received in the measurement chamber 34.

As seen in figure 2, the confinement fluid CF is contained by the confinement housing 27 in a confinement space 77 arranged between the confinement housing 27 on one side and the sleeve 24, the upstream diffuser 20 and the downstream diffuser 22 on the other side.

The confinement fluid CF is transparent to inner structure imaging such as microtomography imaging and/or nuclear magnetic resonance imaging.

The confinement fluid CF is for example water, mineral oil, nitrogen and for example a fluorinated oil.

The confinement frame 72 is for example tubular, the confinement frame being aligned with the rock sample axis A-A'.

The upstream cap 74 comprises a first upstream tubing 78 and preferably comprises a second upstream tubing 80. As shown in figure 2, the upstream cap 74 is for example made of two distinct parts.

The first upstream tubing 78 is fluidically connected to the first upstream opening 40. In particular, the first upstream tubing 78 fluidically connects the first upstream opening 40 to an outside of the measurement cell 12 and in particular fluidically connects the first upstream opening 40 with the fluid regulation arrangement 16 to guide first fluid F1 and/or second fluid F2 into the rock sample 28.

The second upstream tubing 80 is fluidically connected to the second upstream opening 42. In particular, the second upstream tubing 80 fluidically connects the second upstream opening 42 to the outside of the measurement cell 12 and in particular fluidically connects the second upstream opening 42 with the pressure measurement device 44 of the fluid regulation arrangement 16.

The downstream cap 76 comprises a first downstream tubing 82 and a second downstream tubing 84. As shown in figure 2, the downstream cap 76 is for example made of two distinct parts.

The first downstream tubing 82 is fluidically connected to the first downstream opening 62. In particular, the first downstream tubing 82 fluidically connects the first downstream opening 62 to the outside of the measurement cell 12 to evacuate first F1 and/or second F2 fluid from the rock sample 28.

The second downstream tubing 84 is fluidically connected to the second downstream opening 64. In particular, the second downstream tubing 84 fluidically connects the second downstream opening to the outside of the measurement cell 12 to evacuate first fluid F1 from the rock sample 28 through the porous barrier element 18.

One of the upstream 74 and downstream 76 cap comprises a confinement tubing 85, configured for guiding confinement fluid CF into the confinement housing 27. In particular, the confinement tubing 85 opens on the confinement space 77. The confinement tubing 85 connects the fluid regulation arrangement 16 to the confinement space 77.

In the example of figure 2, the downstream cap 76 comprises the confinement tubing 85.

As seen above, the fluid regulation arrangement 16 comprises the pressure measurement device 44. The fluid regulation arrangement 16 also preferably comprises a feeding device 86, a first valve 88 and a second valve 90. The fluid regulation arrangement 16 also comprises a confinement pressuring device 91.

As seen above, the pressure measurement device 44 is fluidically connected with the rock sample 28 and in particular with the inlet face 30 of the rock sample 28.

The pressure measurement device 44 is configured to measure the pressure of the first fluid F1 and/or of the second fluid F2 in the rock sample 28, in particular at the inlet face 30 of the rock sample 28.

The feeding device 86 is configured for feeding the second fluid F2 in the first upstream opening 40, in particular through the first upstream tubing 78.

The feeding device 86 is thus configured for, through the feeding of the second fluid F2 in the first upstream opening 40, to flood the rock sample 28 with the second fluid F2.

As it will be presented later, the feeding device 86 is configured for either feeding the second fluid F2 with a constant flow, or for feeding the second fluid with a constant pressure.

When the feeding device 86 is configured for feeding the second fluid with a constant pressure, the feeding device is for example logically connected with the pressure measurement device 44, the feeding device 86 feeding the second fluid at a flowrate so that the pressure measured by the measuring device is constant.

In the embodiment presented in figures 1 and 2, the feeding device is for example a pump. In a non-illustrated embodiment, the feeding device comprises a centrifuge that is configured for feeding the second fluid F2 in the first upstream opening 40.

The first valve 88 is fluidically connected to the first downstream opening 62. The first valve 88 is configured for selectively freeing or blocking the first downstream opening 62. As presented in figure 1, the measurement system 10 comprises for example a first connection tube 92 connected to the first downstream tubing 82, the first valve 88 being installed on the first connection tube 92 and selectively freeing or blocking the connection tube 92 to free or block the first downstream opening 62.

The second valve 90 is fluidically connected to the second downstream opening 64. The second valve 90 is configured for selectively freeing or blocking the second downstream opening 64. As presented in figure 1, the measurement system 10 comprises for example a second connection tube 94 connected to the second downstream tubing 84, the second valve 90 being installed on the second connection tube 94 and selectively freeing or blocking the connection tube 94 to free or block the second downstream opening 64.

The confinement pressuring device 91 is fluidically connected to the confinement tubing 85. In particular, the confinement pressuring device 91 is configured for pressuring the confinement fluid CF in the confinement housing 27 through the confinement tubing 85.

As illustrated in figure 1, the inner structure fluid imaging device 14 is arranged at an outside of the rock properties measurement cell 12.

The inner structure fluid imaging device 14 is for example arranged around the confinement frame 72.

The inner structure fluid imaging device 14 is configured to evaluate fluids saturation in first fluid F1 and/or second fluid F2 along the rock sample axis A-A'.

The inner structure imaging device 14 is for example a microtomography imaging device or nuclear magnetic resonance imaging device.

A method 100 for preparing a rock sample with a target initial first fluid saturation profile S1iT along a rock sample axis A-A', implemented by the above-described measurement cell 12 and measurement system 10 will now be presented.

As above-mentioned, the first fluid F1 is for example water, such that the initial first fluid saturation S1i is an initial water saturation, also known as Swi, and the second fluid F2 is for example oil.

The method 100 comprises a providing step 110, followed by a primary flooding step 120 with the second fluid F2 and by a secondary flooding step 130 with the second fluid F2.

The method 100 further comprises, for example, prior to the providing step 110, a preliminary flooding step, where the rock sample 28 is completely saturated by the first fluid F1.

The initial first fluid saturation S1i in the rock sample 28 at the providing step 110 is then sensibly equal to 100%, inner cavities of the rock sample being saturated with the first fluid F1 only.

In the providing step 110, the rock sample 28 as above mentioned, saturated in the first fluid F1, is provided.

The primary flooding step 120 with the second fluid F2 comprises feeding the second fluid F2 in the rock sample 28 from the inlet face 30 and retrieving first fluid F1 and/or second fluid F2 from the first open channel 66.

During the primary flooding step 120, second fluid F2 is in particular fed in the rock properties measurement cell 12 by the feeding device 86.

The pressure in first fluid F1 and second fluid F2 is for example measured by the pressure measurement device 44 during the primary flooding step 120.

The flowrate of the second fluid F2 is for example controlled by the feeding device 86 during the primary flooding step. In a specific embodiment, the second fluid F2 is fed in the rock sample with a constant flowrate during the primary flooding. The flowrate of the second fluid F2 is for example controlled by the feeding device 86 to be constant during the primary flooding.

Alternatively, or additionally, the pressure of the second fluid F2 is controlled by the feeding device 86 during the primary flooding step 120. In a specific embodiment, the second fluid is fed in the rock sample with a constant pressure during the primary flooding step 120. The pressure of the second fluid F2 is for example controlled by the feeding device 86 in conjunction with the pressure measurement device 44 so that the pressure of the second fluid F2 is constant during the primary flooding 120.

The primary flooding 120 comprises maintaining the first channel 66 open. The primary flooding comprises for example to that end opening and/or maintaining in an open position the first valve 88 for freeing the first downstream opening 62.

The first fluid F1 and/or the second fluid F2 contained in the rock sample 28 is therefore pressed out of the rock sample by the feeding device 86 and flow freely from the outlet face 32 of the rock sample to be retrieved.

In a specific embodiment, the primary flooding 120 comprises blocking the second channel to close the second channel 68 and prevent any fluid to flow through the second channel 68. The primary flooding 120 comprises for example to that end closing and/or maintaining in a closed position the second valve 90 for blocking the second downstream opening 64.

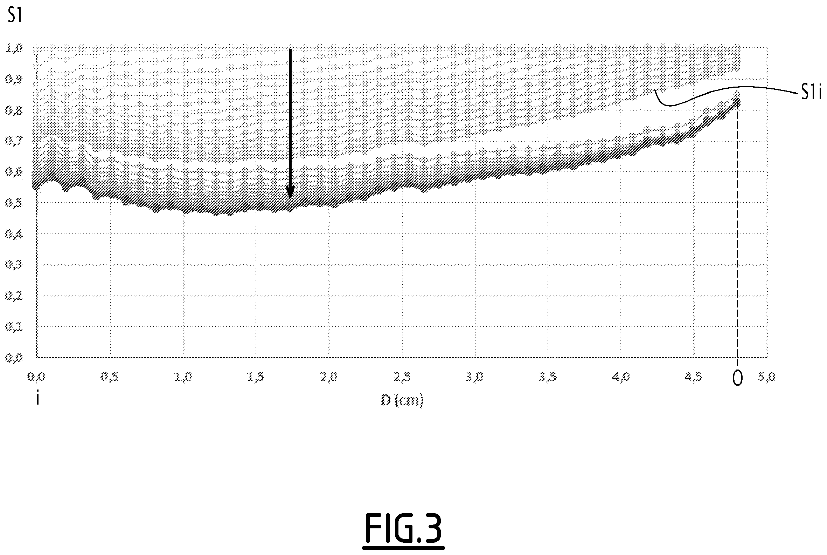

The evolution of the first fluid saturation profile S1i during the primary flooding 120 is illustrated in figure 3. The horizontal axis corresponds to the location along the rock sample axis, from position of the inlet face referenced i in figure 3, to the position of the outlet face, referenced o in figure 3. The vertical axis corresponds to the first fluid saturation S1, the first fluid saturation S1 along the rock sample axis forming at a defined time an initial first fluid saturation profile S1i. The arrow of figure 3 shows the general evolution of the initial first fluid saturation profile S1i during the primary flooding 120.

As visible from figure 3, the first fluid saturation profile S1i is decreasing over the primary flooding step 120 since first fluid F1 is being replaced in the rock sample by second fluid F2 being fed by the feeding device 86.

Once the primary flooding 120 is finished, the primary flooding 120 is stopped and the secondary flooding 130 of the rock sample with the second fluid F2 is started.

In some embodiments, the primary flooding is considered to be finished after a predetermined duration, the primary flooding 120 being stopped and the secondary flooding 130 started after the predetermined duration.

The predetermined duration is for example comprised between 6 hours and 92 hours.

In a preferred embodiment, the primary flooding 120 is stopped and the secondary flooding 130 is started when only second fluid F2 is retrieved from the first channel 66 during the primary flooding 120, that is when no first fluid F1 is being produced from the rock sample 28.

The secondary flooding 130 preferably immediately follows the primary flooding 120.

The secondary flooding step 130 with the second fluid F2 comprises feeding the second fluid F2 in the rock sample 28 from the inlet face 30, and retrieving first fluid F1, from the second channel 68. In particular, since the second channel 68 is connected to the outlet face 32 of the rock sample 28 through the porous barrier element 18 that is permeable to the first fluid F1 and impervious to the second fluid F2, only first fluid F1 is retrieved from the rock sample during the secondary flooding 130.

The second fluid F2 is fed at a start of the secondary flooding 130 at a same pressure as the second fluid F2 is fed at an end of the primary flooding 120. The feeding device 86 is for example configured to feed the second fluid F2 at a same pressure at the end of the primary flooding 120 and at the start of the secondary flooding 130.

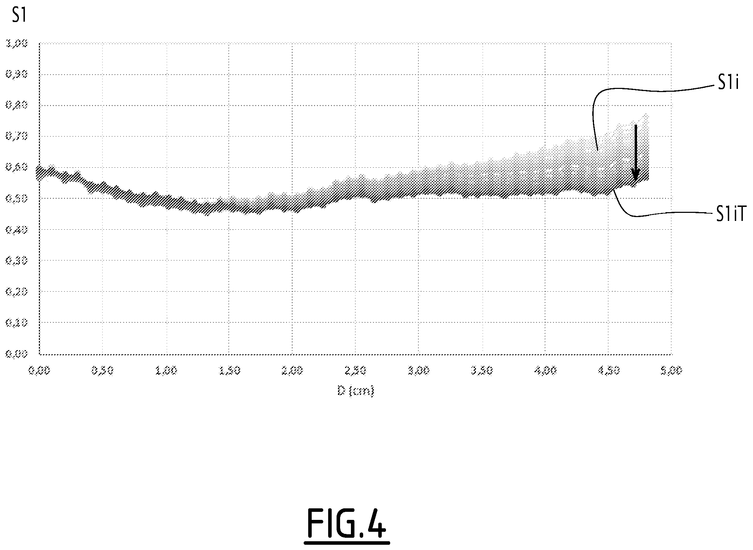

The secondary flooding 130 is configured to obtain the target initial first fluid saturation profile S1iT along the rock sample axis A-A', as shown in figure 4.

The secondary flooding 130 comprises blocking the first channel 66 to close the first channel 66 and prevent any fluid to flow through the first channel 66. The secondary flooding 130 comprises for example to that end closing and/or maintaining in a closed position the first valve 88 for blocking the first downstream opening 62. For example, the primary flooding 120 is stopped and the second flooding 130 is started by said blocking of the first channel 66 to close the first channel 66 and prevent any fluid to flow through the first channel 66.

When the primary flooding 120 comprises blocking the second channel 68, the secondary flooding 130 comprises opening the second channel 68. The secondary flooding 130 comprises for example to that end opening and/or maintaining in an opened position the second valve 90 for freeing the second downstream opening 64.

The second fluid F2 is fed in the rock sample 28 with a constant pressure during the secondary flooding step 130. The pressure of the second fluid F2 is preferably controlled by the feeding device 86 during the secondary flooding step 130.The pressure of the second fluid F2 is for example controlled by the feeding device 86 in conjunction with the pressure measurement device 44 so that the pressure of the second fluid F2 is constant during the secondary flooding 130.

The evolution of the first fluid saturation S1i during the secondary flooding 130 is illustrated in figure 4. The horizontal axis corresponds to the location along the rock sample axis, from position of the inlet face referenced i in figure 4, to the position of the outlet face, referenced in figure 4. The vertical axis corresponds to the first fluid saturation, the first fluid saturation S1 along the rock sample axis forming at a defined time an initial first fluid saturation profile S1i. The arrow of figure 4 shows the general evolution of the first fluid saturation profile S1i during the secondary flooding.

As visible from figure 4, the first fluid saturation profile S1i is further decreasing over the secondary flooding step 130 since first fluid F1 is being replaced in the rock sample by second fluid F2 being fed by the feeding device 86.

As visible from figure 4 the initial first fluid profile S1i along the rock sample axis is homogenized during the secondary flooding step so that the value of the first fluid saturation profile S1 along the rock sample axis A-A' is substantially constant.

Upon reaching the target initial first fluid saturation S1iT, the secondary flooding 130 is finished, the rock sample being finally prepared with the S1iT target initial first fluid saturation profile along the rock sample axis A-A'.

As presented on figure 4, the target initial first fluid saturation profile is for example above 40% along the rock sample axis A-A'.

The first fluid saturation profile S1i along the rock sample axis A-A' is for example monitored by nuclear magnetic resonance imaging during primary flooding and/or secondary flooding. Alternatively, or in complement, a first fluid F1 propagation within the rock sample 28 is for example monitored by microtomography during primary flooding 120 and/or secondary flooding 130.

The first fluid saturation profile S1i along the rock sample axis A-A' and/or the first fluid F1 propagation within the rock sample is in particular monitored with the inner structure imaging device 14.

In some non-described embodiments, the above-mentioned method is implemented with a rock properties measurement cell differing form the above mentioned rock properties measurement cell.

The use water as the first fluid F1 and oil as the second fluid F2 is preferred since these fluids allow simulation of fluids displacements similar as those occurring on an oil exploration site.

Blocking the second channel 68 during the primary flooding allows retrieving without distinction first F1 and/or second fluid F2 through the first channel 66 only.

Feeding second fluid F2 in the rock sample 28 with a constant flowrate during the primary flooding 120 is especially advantageous since it allows a flooding similar to the flooding implemented by the known method of viscous flooding, allowing a fast flooding of the rock sample 28.

Feeding second fluid F2 in the rock sample 28 with a constant pressure during the secondary flooding 130 is especially advantageous since it allows a flooding similar to the flooding implemented by the known method of porous-plate, allowing a fast homogeneous saturation profile of the rock sample 28.

Feeding the second fluid F2 at the start of the secondary flooding 130 at a same pressure as the second fluid is fed F2 at an end of the primary flooding 120 allows not losing capillary pressure in the rock sample 28, allowing thus an homogeneous flooding of the rock sample during the secondary flooding 130.

Stopping the primary flooding 120 and starting the secondary flooding 130 when only second fluid F2 is retrieved from the first channel 66 during the primary flooding 120 allows ensuring a complete primary flooding reduces as much as possible the time to reach the target initial first fluid saturation profile S1iT.

An upstream face 52 of the porous barrier element 18 being placed in contact with the outlet face 32, allows a capillary contact between the rock sample 28 and the porous plate 18, ensuring a proper flow of the first fluid F1 through the barrier element 18 when the first channel 66 is blocked and the second channel 68 is open.

## Claims

1. Method (100) for preparing a rock sample (28) with a target initial first fluid saturation profile along a rock sample axis (A-A'), the rock sample (28) comprising an inlet face (30) and an outlet face (32), the inlet face (30) and the outlet face (32) being opposed relatively to the rock sample axis (A-A') and being transverse to the rock sample axis (A-A'), the method (100) comprising the successive steps of :
- providing (110) a rock sample (28) saturated in a first fluid (F1);
- primary flooding (120) of the rock sample (28) with a second fluid (F2), comprising:
+ feeding the second fluid (F2) in the rock sample (28) from the inlet face (30), and
+ retrieving the first fluid (F1) and/or the second fluid (F2) from a first open channel (66) directly connected to the outlet face (32) of the rock sample (28);
- secondary flooding (130) of the rock sample (28) with the second fluid (F2), to obtain the target initial first fluid saturation profile along the rock sample axis (A-A'), comprising:
+ blocking the first channel (66) to close the first channel (66) and prevent any fluid to flow through the first channel (66);
+ feeding the second fluid (F2) in the rock sample (28) from the inlet face (30), and
+ retrieving the first fluid (F2) from a second channel (68) connected to the outlet face (32) of the rock sample (28) through a porous barrier element (18), the porous barrier element (18) being permeable to the first fluid (F1) and impervious to the second fluid (F2).

2. Method (100) according to claim 1, wherein the first fluid (F1) is water and/or the second fluid (F2) is oil.

3. Method (100) according to claim 1 or 2, wherein the primary flooding (120) comprises blocking the second channel (68) to close the second channel (68) and prevent any fluid to flow through the second channel (68).

4. Method (100) according to any one of the preceding claims, wherein the target initial first fluid saturation profile along the rock sample axis (A-A') is obtained by controlling a second fluid (F2) flowrate and/or pressure during the primary flooding (120) and/or the secondary flooding (130).

5. Method (100) according to claim 4, wherein the second fluid (F2) is fed in the rock sample (28) with a constant flowrate during the primary flooding (120).

6. Method (100) according to claims 4 or 5, wherein the second fluid (F2) is fed in the rock sample (28) with a constant pressure during the primary flooding (120).

7. Method (100) according to any one of the claims 4 to 6, wherein the second fluid (F2) is fed in the rock sample (28) with a constant pressure during the secondary flooding (130).

8. Method (100) according to any one of the preceding claims, wherein the secondary flooding (130) immediately follows the primary flooding (120), the second fluid (F2) being fed at a start of the secondary flooding (130) at a same pressure as the second fluid (F2) is fed at an end of the primary flooding (120).

9. Method (100) according to any one of the preceding claims, wherein the primary flooding (120) is stopped and the secondary flooding (130) is started when only the second fluid (F2) is retrieved from the first channel (66) during the primary flooding (120).

10. Method (100) according to any one of the preceding claims, wherein the first fluid (F1) saturation profile along the rock sample axis (A-A') is monitored by nuclear magnetic resonance imaging during primary flooding (120) and/or secondary flooding (130).

11. Method (100) according to any one of the preceding claims, wherein the target initial first fluid (F1) saturation profile is above 40% along the rock sample axis (A-A') and/or the rock sample (28) permeability is under 50mD.

12. Method (100) according to any one of the preceding claims, wherein an upstream face (52) of the porous barrier element (18) is placed in contact with the outlet face (32), the first channel (66) passing through the porous barrier element (18), the second channel (18) opening facing a downstream face (54) of the porous barrier element (18).

## Patentansprüche

1. Verfahren (100) zum Vorbereiten einer Gesteinsprobe (28) mit einem angestrebten Anfangssättigungsprofil eines ersten Fluids entlang einer Gesteinsprobenachse (A-A'), wobei die Gesteinsprobe (28) eine Einlassseite (30) und eine Auslassseite (32) umfasst, wobei die Einlassseite (30) und die Auslassseite (32) in Bezug auf die Gesteinsprobenachse (A-A') gegenüberliegen und quer zur Gesteinsprobenachse (A-A') verlaufen, wobei das Verfahren (100) die folgenden aufeinanderfolgenden Schritte umfasst:
- Bereitstellen (110) einer in einem ersten Fluid (F1) gesättigten Gesteinsprobe (28);
- primäre Flutung (120) der Gesteinsprobe (28) mit einem zweiten Fluid (F2), umfassend:
+ Einspeisen des zweiten Fluids (F2) in die Gesteinsprobe (28) von der Einlassseite (30) aus und
+ Wiedergewinnen des ersten Fluids (F1) und/oder des zweiten Fluids (F2) aus einem ersten offenen Kanal (66), der unmittelbar mit der Auslassseite (32) der Gesteinsprobe (28) verbunden ist;
- sekundäre Flutung (130) der Gesteinsprobe (28) mit dem zweiten Fluid (F2), um das angestrebte Anfangssättigungsprofil des ersten Fluids entlang der Gesteinsprobenachse (A-A') zu erhalten, umfassend:
+ Blockieren des ersten Kanals (66), um den ersten Kanal (66) zu verschließen und zu verhindern, dass Fluid durch den ersten Kanal (66) fließt;
+ Einspeisen des zweiten Fluids (F2) in die Gesteinsprobe (28) von der Einlassseite (30) aus und
+ Wiedergewinnen des ersten Fluids (F2) aus einem zweiten Kanal (68), der mit der Auslassseite (32) der Gesteinsprobe (28) durch ein poröses Barriereelement (18) verbunden ist, wobei das poröse Barriereelement (18) für das erste Fluid (F1) durchlässig und für das zweite Fluid (F2) undurchlässig ist.

2. Verfahren (100) nach Anspruch 1, wobei das erste Fluid (F1) Wasser und/oder das zweite Fluid (F2) Öl ist.

3. Verfahren (100) nach Anspruch 1 oder 2, wobei die primäre Flutung (120) das Blockieren des zweiten Kanals (68) umfasst, um den zweiten Kanal (68) zu verschließen und zu verhindern, dass Fluid durch den zweiten Kanal (68) fließt.

4. Verfahren (100) nach einem der vorhergehenden Ansprüche, wobei das angestrebte Anfangssättigungsprofil des ersten Fluids entlang der Gesteinsprobenachse (A-A') durch Steuern einer Flussrate und/oder eines Drucks eines zweiten Fluids (F2) während der primären Flutung (120) und/oder der sekundären Flutung (130) erhalten wird.

5. Verfahren (100) nach Anspruch 4, wobei das zweite Fluid (F2) während der primären Flutung (120) mit einer konstanten Flussrate in die Gesteinsprobe (28) eingespeist wird.

6. Verfahren (100) nach Anspruch 4 oder 5, wobei das zweite Fluid (F2) während der primären Flutung (120) mit einem konstanten Druck in die Gesteinsprobe (28) eingespeist wird.

7. Verfahren (100) nach einem der Ansprüche 4 bis 6, wobei das zweite Fluid (F2) während der sekundären Flutung (130) mit einem konstanten Druck in die Gesteinsprobe (28) eingespeist wird.

8. Verfahren (100) nach einem der vorhergehenden Ansprüche, wobei die sekundäre Flutung (130) unmittelbar auf die primäre Flutung (120) folgt, wobei das zweite Fluid (F2) bei einem Start der sekundären Flutung (130) mit demselben Druck eingespeist wird, mit dem das zweite Fluid (F2) bei einem Ende der primären Flutung (120) eingespeist wird.

9. Verfahren (100) nach einem der vorhergehenden Ansprüche, wobei die primäre Flutung (120) gestoppt wird und die sekundäre Flutung (130) gestartet wird, wenn während der primären Flutung (120) nur das zweite Fluid (F2) aus dem ersten Kanal (66) wiedergewonnen wird.

10. Verfahren (100) nach einem der vorhergehenden Ansprüche, wobei das Sättigungsprofil des ersten Fluids (F1) entlang der Gesteinsprobenachse (A-A') während der primären Flutung (120) und/oder der sekundären Flutung (130) durch Bilderzeugung mittels magnetischer Kernresonanz überwacht wird.

11. Verfahren (100) nach einem der vorhergehenden Ansprüche, wobei das angestrebte Anfangssättigungsprofil des ersten Fluids (F1) entlang der Gesteinsprobenachse (A-A') über 40 % liegt und/oder die Durchlässigkeit der Gesteinsprobe (28) unter 50 mD liegt.

12. Verfahren (100) nach einem der vorhergehenden Ansprüche, wobei eine stromaufwärtige Seite (52) des porösen Barriereelements (18) in Kontakt mit der Auslassseite (32) platziert ist, wobei der erste Kanal (66) durch das poröse Barriereelement (18) verläuft und die Öffnung des zweiten Kanals (18) einer stromabwärtigen Seite (54) des porösen Barriereelements (18) zugewandt ist.

## Revendications

1. Procédé (100) de préparation d'un échantillon de roche (28) avec un profil de saturation de premier fluide initial cible le long d'un axe d'échantillon de roche (A-A'), l'échantillon de roche (28) comprenant une face d'entrée (30) et une face de sortie (32), la face d'entrée (30) et la face de sortie (32) étant opposées par rapport à l'axe d'échantillon de roche (A-A') et étant transversales à l'axe d'échantillon de roche (A-A'), le procédé (100) comprenant les étapes successives suivantes :
- la fourniture (110) d'un échantillon de roche (28) saturé dans un premier fluide (F1) ;
- le noyage primaire (120) de l'échantillon de roche (28) avec un deuxième fluide (F2), comprenant :
+ l'introduction du deuxième fluide (F2) dans l'échantillon de roche (28) à partir de la face d'entrée (30), et
+ la récupération du premier fluide (F1) et/ou du deuxième fluide (F2) à partir d'un premier canal ouvert (66) directement relié à la face de sortie (32) de l'échantillon de roche (28) ;
- le noyage secondaire (130) de l'échantillon de roche (28) avec le deuxième fluide (F2), pour obtenir le profil de saturation de premier fluide initial cible le long de l'axe d'échantillon de roche (A-A'), comprenant :
+ le blocage du premier canal (66) pour fermer le premier canal (66) et empêcher tout fluide de s'écouler à travers le premier canal (66) ;
+ l'introduction du deuxième fluide (F2) dans l'échantillon de roche (28) à partir de la face d'entrée (30), et
+ la récupération du premier fluide (F2) à partir d'un deuxième canal (68) relié à la face de sortie (32) de l'échantillon de roche (28) à travers un élément de barrière poreux (18), l'élément de barrière poreux (18) étant perméable au premier fluide (F1) et imperméable au deuxième fluide (F2).

2. Procédé (100) selon la revendication 1, dans lequel le premier fluide (F1) est de l'eau et/ou le deuxième fluide (F2) est de l'huile.

3. Procédé (100) selon la revendication 1 ou 2, dans lequel le noyage primaire (120) comprend le blocage du deuxième canal (68) pour fermer le deuxième canal (68) et empêcher tout fluide de s'écouler à travers le deuxième canal (68).

4. Procédé (100) selon l'une quelconque des revendications précédentes, dans lequel le profil de saturation de premier fluide initial cible le long de l'axe d'échantillon de roche (A-A') est obtenu en contrôlant le débit et/ou la pression d'un deuxième fluide (F2) pendant le noyage primaire (120) et/ou le noyage secondaire (130).

5. Procédé (100) selon la revendication 4, dans lequel le deuxième fluide (F2) est introduit dans l'échantillon de roche (28) avec un débit constant pendant le noyage primaire (120).

6. Procédé (100) selon les revendications 4 ou 5, dans lequel le deuxième fluide (F2) est introduit dans l'échantillon de roche (28) avec une pression constante pendant le noyage primaire (120).

7. Procédé (100) selon l'une quelconque des revendications 4 à 6, dans lequel le deuxième fluide (F2) est introduit dans l'échantillon de roche (28) avec une pression constante pendant le noyage secondaire (130).

8. Procédé (100) selon l'une quelconque des revendications précédentes, dans lequel le noyage secondaire (130) suit immédiatement le noyage primaire (120), le deuxième fluide (F2) étant introduit au début du noyage secondaire (130) à une même pression que celle à laquelle le deuxième fluide (F2) est introduit à la fin du noyage primaire (120).

9. Procédé (100) selon l'une quelconque des revendications précédentes, dans lequel le noyage primaire (120) est arrêté et le noyage secondaire (130) démarre lorsque seul le deuxième fluide (F2) est récupéré à partir du premier canal (66) pendant le noyage primaire (120).

10. Procédé (100) selon l'une quelconque des revendications précédentes, dans lequel le profil de saturation de premier fluide (F1) le long de l'axe d'échantillon de roche (A-A') est surveillé par imagerie par résonance magnétique nucléaire pendant le noyage primaire (120) et/ou le noyage secondaire (130).

11. Procédé (100) selon l'une quelconque des revendications précédentes, dans lequel le profil de saturation de premier fluide (F1) initial cible est supérieur à 40 % le long de l'axe d'échantillon de roche (A-A') et/ou la perméabilité de l'échantillon de roche (28) est inférieure à 50 mD.

12. Procédé (100) selon l'une quelconque des revendications précédentes, dans lequel une face amont (52) de l'élément de barrière poreux (18) est placée en contact avec la face de sortie (32), le premier canal (66) traversant l'élément de barrière poreux (18), le deuxième canal (18) s'ouvrant face à une face aval (54) de l'élément de barrière poreux (18).
